# EUROPÄISCHE PATENTSCHRIFT

(11) **EP 2 170 184 B1**
(45) Veröffentlichungstag und Bekanntmachung des Hinweises auf die Patenterteilung: **03.04.2013**
(21) Anmeldenummer: 08773823.3
(22) Anmeldetag: 03.07.2008
(51) Int. Cl.: A61B 17/32, A61B 17/29, A61B 19/00, A61B 17/16

(54) **CHIRURGISCHES INSTRUMENT**
SURGICAL INSTRUMENT
INSTRUMENT CHIRURGICAL

(30) Priorität: 13.07.2007 DE 202007009929 U
(43) Veröffentlichungstag der Anmeldung: 07.04.2010
(73) Patentinhaber: Schilling, Hermann, 78603 Renquishausen (DE)
(72) Erfinder: Schilling, Hermann, 78603 Renquishausen (DE)
(74) Vertreter: Daub, Thomas
(86) Internationale Anmeldenummer: PCT/EP2008/005411
(87) Internationale Veröffentlichungsnummer: WO 2009/010192

(56) Entgegenhaltungen:
- EP-A- 1 790 292
- US-A- 2 682 414
- US-A- 5 297 538
- US-A1- 2004 044 346
- US-A1- 2006 074 432

## Beschreibung

### Stand der Technik

Die Erfindung geht insbesondere aus von einem chirurgischen Instrument nach dem Oberbegriff des Anspruchs 1.

Aus der DE 199 49 422 A1 ist ein chirurgisches Instrument mit einer ersten, feststehenden und einer zweiten bewegbaren Stegeinheit und mit einem ersten, feststehenden, mit der ersten Stegeinheit einstückig ausgeführten Griffmittel und einem relativ zum ersten Griffmittel bewegbaren zweiten Griffmittel, das über eine Langlochverbindung mit der zweiten Stegeinheit gekoppelt ist, bekannt. An die Stegeinheiten sind von Schneidköpfen gebildete Funktionsmittel angeformt.

Aus der US 2006/0074432 A1 ist ein chirurgisches Instrument mit einer ersten und einer zweiten Stegeinheit, die jeweils ein Funktionsmittel aufweisen und die relativ zueinander bewegbar gelagert sind, und mit einem ersten und einem zweiten Griffmittel, die relativ zueinander bewegbar gelagert und zur Betätigung der Stegeinheit vorgesehen sind, wobei die Stegeinheiten ein lösbares Funktionsmittel aufweisen, und mit einem Funktionsmittelbefestigungsmechanismus, der ein kippbar gelagertes Befestigungsmittel umfasst, bekannt. Der Funktionsmittelbefestigungsmechanismus ist im Wesentlicher zur Übertragung von Querkräften vorgesehen, die entstehen, wenn die beiden Stegeinheiten und Funktionsmittel voneinander wegbewegt werden, um z.B. eine Operationsöffnung aufzuspreizen.

Befestigungselemente der Funktionsmittel sind in Ausnehmungen der Stegeinheiten in Richtung einer Einführrichtung einführbar, wobei Einführschrägen der Befestigungselemente der Funktionsmittel vorgesehen sind. Werden die Befestigungselemente der Funktionsmittel aufgrund eines Zusammendrückens, welches durch jeweils einen Aktivierungs-Knopf eines Befestigungsmechanismus ermöglicht wird, vcn den zwei Enden der Befestigungselemente der Funktionsmittel, welche jeweils den Befestigungsmittel gegenüberliegen und welche beim Zusammendrücken ein Federmittel komprimierer, unzureichend weit auseinander bewegt, werden sie aufgrund des Einführens der Befestigungselemente der Funktionsmittel auseinander gedrückt, bis die Funktionsmittel eine Endstellung erreichen, in welcher die Befestigungsmittel, wenn die Aktivierungs-Knöpfe von einem Benutzer freigegeben werden, wieder aufgrund einer Kraft der komprimierten Federmittel, in ihre Ausgangsstellung vor den Zusammendrücken zurückkehren. Die Befestigungsmechanlsmen weisen dabei jeweils ein Befestigungselement in Form eines Hakens auf.

Aus der US-A-5 297 538 ist ein chirurgisches Instrument mit einer ersten und einer zweiten Stegeinheit, die jeweils ein Funktionsmittel aufweisen und die relativ zueinander bewegbar gelagert sind, und mit einem ersten und einem zweiten Griffmittel, die relativ zueinander bewegbar gelagert und zur Be tätigunq der Stegeinheit vorgesehen sind, wobei die Stegeinheiten ein lösbares Funktionomittel aufweisen, und mit jeweils einem Funktionsmittelbefestigungsmechanismus, der jeweils ein kippbar gelagertes Befestigungsmittel umfasst, bekannt. Die Funktionsmittel werden auf Stegteile aufgeschoben, wobei die Befestigurgsmittel eine Rastfunktion aufweisen, welche eine Vielzahl von möglichen Positionen der Funktionsmittel entlang der Stegteile zulässt. Der Funktionsmittelbefestigungsmechanismus ist nicht zur Übertragung von Zug- und Druckbewegungen vorgesehen.

Der Erfindung liegt insbesondere die Aufgabe zugrunde, ein gattungsgemäßes chirurgisches Instrument bereitzustellen, welches bei einer kostengünstigen Herstellung ein komfortables Auswechseln der Funktionsmittel erlaubt, und welches insbesondere die Übertragung von Zug- und Druckbewegungen erlaubt. Sie wird gemäß der Erfindung durch die Merkmale des Patentanspruchs 1 gelöst. Weitere Ausgestaltungen ergeben sich aus den Unteransprüchen.

### Vorteile der Erfindung

Vorgeschlagen wird ein chirurgisches Instrument mit einer ersten und zumindest einer zweiten Stegeinheit, die jeweils wenigstens ein Funktionsmittel aufweisen und die relativ zueinander bewegbar gelagert sind, und mit einem ersten und einem zweiten Griffmittel, die relativ zueinander bewegbar gelagert und zur Betätigung der Stegeirheiten vorgesehen sind, wobei zumindest eine Stegeinheit wenigstens ein lösbares Funktionsmittel aufweist, und mit zumindest einem Funktionsmittelbefestigungsmechanismus, der wenigstens ein kippbar gelagertes Befestigungsmittel umfasst, wobei die Funktionsmittelbefestigungsmechanismen zum Übertragen von Zug- und Druckbewegungen vorgesehen sind und die obere Stegeinheit relativ zu der unteren Stegeinheit translatorisch bewegbar gelagert ist, wobei Befestigungselemente der Stegteile in Ausnehmungen der Stegteile in Richtung einer Einführrichtung einführbar sind, wobei Einführschrägen der Stegteile vorgesehen sind, wobei wenn die Befestigungselemente aufgrund eines Zusammendrückens, welches durch jeweils eine Ausnehmung in den Stegteilen ermöglicht wird, von den zwei Enden der Befestigungsmittel, welche jeweils den Befestigungselsmenten gegenüberliegen und welche bei dem Zusammendrücken ein Federmittel komprimieren, unzureichend weit auseinander bewegt sind, sie aufgrund des Einführens der Stegteile in die Ausnehmungen bei dem Berühren der Einführschrägen weiter auseinander gedrückt werden, bis die Stegteile bei dem Einführen eine Endstellung erreichen, in welcher die Befestigungs-mittel, wenn die komprimierten Enden der Befestigungsmittel von einem Benutzer freigegeben werden, wieder aufgrund einer Kraft des komprimierten Federmittels, welches ein Teil der Funktionsmittelbefestigungsmechanismen ist, in ihre Ausgangsstellung vor dem Zusammendrücken durch einen Benutzer zurückkehren, wobei die Funktionsmittelbefestigungsmechanismen jeweils wenigstens ein Befestigungselement umfassen, welches als Verzahnung und/oder Kamm ausgebildet ist, Unter "vorgesehen" soll dabei insbesondere speziell ausgestattet und/oder ausgelegt verstanden werden. Unter einem "Funktionsmittel" soll dabei insbesondere ein Mittel verstanden werden, welches dazu vorgesehen ist, während der Durchführung einer Behandlungsfunktion bei einem In-Kontakt-Treten mit einem menschlichen und/oder tierischen Körperteil Verwendung zu finden, und insbesondere soll darunter ein Trennmittel und/oder ein Greifmittel verstanden werden. Unter einem "Funktionsmittelbefestigungsmechanismus" soll insbesondere ein Befestigungsmechanismus verstanden werden, welcher dazu vorgesehen ist, das Funktionsmittel und/oder eine mit dem Funktionsmittel verbundene und/oder verbindbare Stegspitze und/oder ein Stegvorderteil und/oder ein Stegteil an einem Stegteil zu befestigen. Mit einer erfindungsgemäßen Ausgestaltung kann ein einfaches und komfortables Befestigen und Ablösen des Funktionsmittels bei einer stabilen Befestigung des Funktionsmittels erreicht werden.

Mit Vorteil weist die Verzahnung wenigstens einen Zahn mit wenigstens zwei relativ zu einer sich senkrecht zu einer Einführrichtung erstreckenden Ebene geneigten Flächen auf. Mit einer erfindungsgemäßen Ausgestaltung kann eine besonders stabile Befestigung zum Übertragen von Zug- und Druckbewegungen erreicht werden.

Vorzugsweise weist wenigstens eine Stegeinheit zumindest ein lösbares Stegteil auf. Hiermit kann eine flexible Verwendbarkeit des chirurgischen Instruments erreicht werden.

### Zeichnung

Weitere Vorteile ergeben sich aus der folgenden Zeichnungsbeschreibung. In der Zeichnung sind Ausführungsbeispiele der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein chirurgisches Instrument in einer Seitenansicht,
- Fig. 2: einen Teil des chirurgischen Instruments in einer Draufsicht,
- Fig. 3: ein alternatives Ausführungsbeispiel eines Befestigungsmittels und
- Fig. 4: alternative Ausführungsbeispiele zweier Stegteile mit Funktionsmitteln.

### Beschreibung des Ausführungsbeispiels

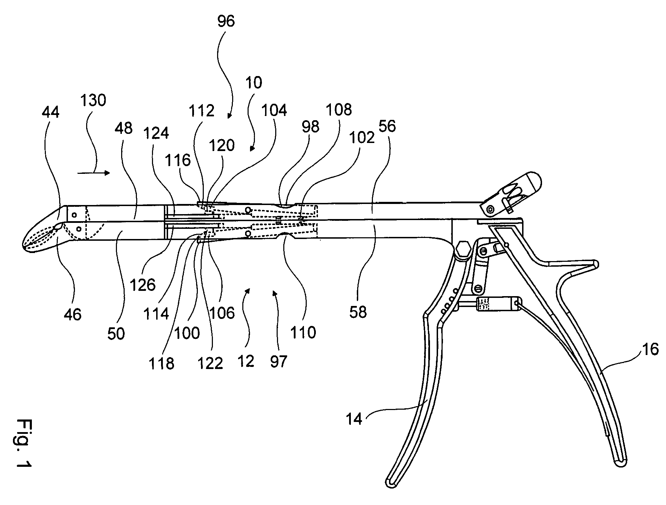

Figur 1 zeigt ein chirurgisches Instrument, und zwar eine so genannte Knochenzange. Das chirurgische Instrument weist zwei Stegeinheiten 10, 12 auf, welche jeweils ein Funktionsmittel 44, 46 umfassen. In dem vorliegenden Fall ist das Funktionsmittel als Trennmittel ausgebildet. Das Funktionsmittel kann alternativ als ein weiteres, dem Fachmann als sinnvoll erscheinendes Mittel zur Ausführung einer weiteren Funktion ausgebildet sein. Mit Ausnahme des Funktionsmittels 44 ist die obere Stegeinheit 10 relativ zu der unteren Stegeinheit 12 translatorisch bewegbar gelagert. Ferner weist das chirurgische Instrument ein erstes und ein zweites Griffmittel 14, 16 auf. Das erste Griffmittel 14 ist schwenkbar an der zweiten Stegeinheit 12 gelagert und ist damit relativ zu dem zweiten Griffmittel 16, das über eine nicht näher dargestellte Schraubverbindung mit der zweiten Stegeinheit 12 starr verbunden ist, bewegbar gelagert. Die Griffmittel 14, 16 sind zur Führung des chirurgischen Instruments und zur Betätigung der Stegeinheiten 10, 12 bzw. zum Bewegen der ersten Stegeinheit 10 relativ zu der zweiten Stegeinheit 12 vorgesehen. Die beiden Griffmittel 14, 16 und die beiden Stegeinheiten 10, 12 sind jeweils von separaten Bauteilen gebildet. Bei einer translatorischen Bewegung der Stegeinheit 10 relativ zu der Stegeinheit 12 führt das Funktionsmittel 44 relativ zu der unteren Stegeinheit 12 und zu dem Funktionsmittel 46 eine Drehbewegung durch.

Die obere Stegeinheit 10 weist zwei Stegteile 48, 56 auf. Ferner weist die untere Stegeinheit 12 zwei Stegteile 50, 58 auf. Die Stegteile 48, 56 sind mittels eines Funktionsmittelbefestigungsmechanismus 96 aneinander befestigbar und voneinander trennbar. Die Stegteile 50, 58 sind ebenfalls mittels eines Funktionsmittelbefestigungsmechanismus 97 aneinander befestigbar und voneinander trennbar. Mittels der Funktionsmittelbefestigungsmechanismen 96, 97 sind die Funktionsmittel 44, 46 von den Stegteilen 56, 58 lösbar. Die Funktionsmittelbefestigungsmechanismen 96, 97 weisen mit den Stegteilen 48, 50, 56, 58 jeweils ein kippbar gelagertes Befestigungsmittel 98, 100 auf. Das Befestigungsmittel 98 ist ein Teil des Stegteils 56. Analog ist das Befestigungsmittel 100 ein Teil des Stegteils 58. Die Befestigungsmittel 98, 100 sind relativ zu den anderen Teilen der Stegteile 56, 58 kippbar gelagert. Die Befestigungsmittel 98, 100 weisen an einem ihrer Enden jeweils ein als Haken ausgebildetes Befestigungselement 116, 118 auf, welche bei einem Kippen der Befestigungsmittel 98, 100 relativ zu den Stegteilen 56, 58 auseinander bewegt werden. Ferner weisen die Stegteile 48, 50 jeweils ein als Haken ausgebildetes Befestigungselement 120, 122 auf.

Um mittels der Funktionsmittelbefestigungsmechanismen 96, 97 die Stegteile 48, 50, welche die Funktionsmittel 44, 46 aufweisen, an den Stegteilen 56, 58 zu befestigen, werden die Befestigungselemente 120, 122 der Stegteile 48, 50 in Ausnehmungen 124, 126 der Stegteile 56, 58 in Richtung einer Einführrichtung 130 eingeführt. Dieser Vorgang wird durch Einführschrägen 112, 114 der Stegteile 56, 58, auf welche die Befestigungselemente 120, 122 je nach Kippzustand der Befestigungsmittel 98, 100 treffen können, unterstützt. Sind die Befestigungselemente 120, 122 aufgrund eines Zusammendrückens, welches durch jeweils eine Ausnehmung 108, 110 in den Stegteilen 56, 58 ermöglicht wird, von zwei Enden der Befestigungsmittel 98, 100, welche jeweils den Befestigungselementen 120, 122 gegenüberliegen und welche bei dem Zusammendrücken ein Federmittel 102 komprimieren, unzureichend weit auseinander bewegt, so werden sie aufgrund des Einführens der Stegteile 48, 50 in die Ausnehmungen 124, 126 bei dem Berühren der Einführschrägen 112, 114 weiter auseinandergedrückt, bis die Stegteile 48, 50 bei dem Einführen eine Endstellung erreichen, in welcher die Befestigungsmittel 98, 100, wenn die komprimierten Enden der Befestigungsmittel 98, 100 von einem Benutzer freigegeben werden, wieder aufgrund einer Kraft des komprimierten Federmittels 102, welches ein Teil der Funktionsmittelbefestigungsmechanismen 96, 97 ist, in ihre Ausgangsstellung vor dem Zusammendrücken durch einen Benutzer zurückkehren. Nach dem Zurückkehren der Befestigungsmittel 98, 100 in die Ausgangsstellung greifen die Befestigungselemente 116, 120 und die Befestigungselemente 118, 120 ineinander ein. Dabei greifen die Befestigungselemente 120, 122 in Ausnehmungen 104, 106 der Befestigungsmittel 98, 100 ein. Ein Entfernen der Stegteile 48, 50 von den Stegteilen 56, 58 kann durch ein abermaliges Zusammendrücken der Befestigungsmittel 98, 100 erfolgen.

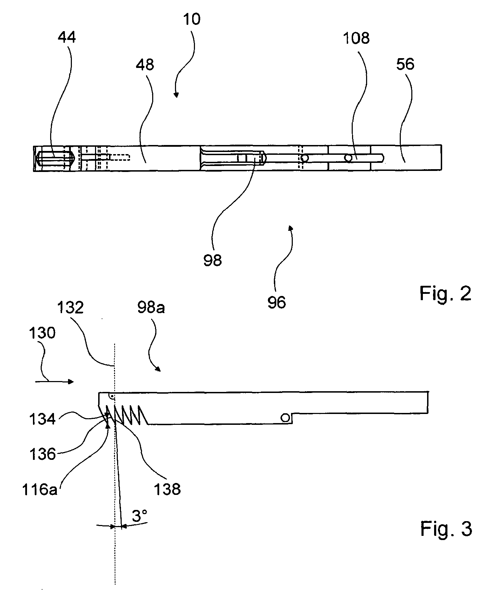

Figur 2 zeigt eine Draufsicht auf den Funktionsmittelbefestigungsmechanismus 96. Das Befestigungsmittel 98 ist senkrecht zu einer Haupterstreckungsrichtung des Stegteils 56 mittig an dem Stegteil 56 angeordnet.

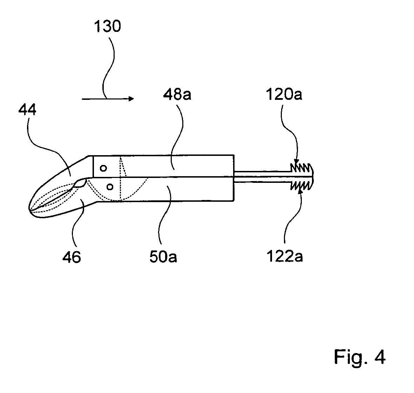

In den Figuren 3 und 4 sind alternative Ausführungsbeispiele dargestellt. Im Wesentlichen gleich bleibende Bauteile, Merkmale und Funktionen sind grundsätzlich mit den gleichen Bezugszeichen beziffert. Zur Unterscheidung der Ausführungsbeispiele sind jedoch den Bezugszeichen der Ausführungsbeispiele in den Figuren 3 und 4 der Buchstaben "a" hinzugefügt. Die nachfolgende Beschreibung beschränkt sich im Wesentlichen auf die Unterschiede zu dem Ausführungsbeispiel in der Figur 1, wobei bezüglich gleich bleibender Bauteile, Merkmale und Funktionen auf die Beschreibung des Ausführungsbeispiels in der Figur 1 verwiesen werden kann.

Figur 3 zeigt ein alternatives Ausführungsbeispiel eines Befestigungsmittels 98a. Eine Haupterstreckungsrichtung des Befestigungsmittels 98a ist parallel zu der Einführrichtung 130. An einem Ende weist das Befestigungsmittel 98a ein Befestigungselement 116a auf, welches als Verzahnung ausgebildet ist. Das Befestigungselement 116a umfasst einzelne Zähne 134. Die Zähne 134 umfassen jeweils zwei relativ zu einer sich senkrecht zu der Einführrichtung 130 erstreckenden Ebene 132 geneigte Flächen 136, 138. Die Fläche 138 schließt mit der Ebene 132 einen 3° großen Winkel ein.

Figur 4 zeigt ein alternatives Ausführungsbeispiel von Stegteilen 48a, 50a, welche dazu vorgesehen sind, in Kombination mit dem Befestigungsmittel 98a und einem zu dem Befestigungsmittel 98a analog ausgebildeten Befestigungsmittel 100a, welches ein alternatives Ausführungsbeispiel des Befestigungsmittels 100 (Figur 1) ist, verwendet zu werden. Die Stegteile 48a, 50a umfassen jeweils an einem ihrer Enden, welche in montiertem Zustand in die Ausnehmungen 124, 126 (Figur 1) eingeführt sind, Befestigungselemente 120a, 122a, welche als Verzahnungen ausgebildet sind und komplementär zu dem Befestigungselement 116a und einem dazu analog ausgebildeten Befestigungselement 118a, das nicht näher dargestellt ist, sind. In dem montierten Zustand greifen die als Verzahnungen ausgebildeten Befestigungselemente 120a, 122a in die als Verzahnung ausgebildeten Befestigungselemente 116a, 118a lückenlos ein, womit eine stabile Verbindung der Stegteile 48a, 50a mit den Befestigungsmitteln 98a, 100a gebildet wird.

### Bezugszeichen

- 10: Stegeinheit
- 12: Stegeinheit
- 14: Griffmittel
- 16: Griffmittel
- 44: Funktionsmittel
- 46: Funktionsmittel
- 48: Stegteil
- 50: Stegteil
- 56: Stegteil
- 58: Stegteil
- 96: Funktionsmittelbefestigungsmechanismus
- 97: Funktionsmittelbefestigungsmechanismus
- 98: Befestigungsmittel
- 100: Befestigungsmittel
- 102: Federmittel
- 104: Ausnehmung
- 106: Ausnehmung
- 108: Ausnehmung
- 110: Ausnehmung
- 112: Einführschräge
- 114: Einführschräge
- 116: Befestigungselement
- 118: Befestigungselement
- 120: Befestigungselement
- 122: Befestigungselement
- 124: Ausnehmung
- 126: Ausnehmung
- 130: Einführrichtung
- 132: Ebene
- 134: Zahn
- 136: Fläche
- 138: Fläche

## Patentansprüche

1. Chirurgisches Instrument mit einer ersten und zumindest einer zweiten Stegeinheit (10, 12), die jeweils zwei Stegteile (48, 56, 50, 58) und wenigstens ein Funktionsmittel (44, 46) aufweisen und die relativ zueinander bewegbar gelagert sind, und mit einem ersten und einem zweiten Griffmittel (14, 16), die relativ zueinander bewegbar gelagert und zur Betätigung der Stegeinheiten (10, 12) vorgesehen sind, wobei die zwei Stegeinheiten (10, 12) jeweils ein lösbares Funktionsmittel (44, 46) aufweisen, und mit jeweils einem Funktionsmittelbefestigungsmechanismus (96, 97), der jeweils wenigstens ein kippbar gelagertes Befestigungsmittel (98, 100) umfasst, wobei die Funktionsmittelbefestigungsmechanismen (96, 97) zum Übertragen von Zug- und Druckbewegungen vorgesehen sind und die obere Stegeinheit (10) relativ zu der unteren Stegeinheit (12) translatorisch bewegbar gelagert ist, wobei Befestigungselemente (120, 122) der Stegteile (48, 50) in Ausnehmungen (124, 126) der Stegteile (56, 58) in Richtung einer Einführrichtung (130) einführbar sind, wobei Einführschrägen (112, 114) der Stegteile (56, 58) vorgesehen sind, wobei wenn die Befestigungselemente (120, 122) aufgrund eines Zusammendrückens, welches durch jeweils eine Ausnehmung (108, 110) in den Stegteilen (56, 58) ermöglicht wird, von den zwei Enden der Befestigungsmittel (98, 100), welche jeweils den Befestigungselementen (120, 122) gegenüberliegen und welche bei dem Zusammendrücken ein Federmittel (102) komprimieren, unzureichend weit auseinander bewegt sind, sie aufgrund des Einführens der Stegteile (48, 50) in die Ausnehmungen (124, 126) bei dem Berühren der Einführschrägen (112, 114) weiter auseinander gedrückt werden, bis die Stegteile (48, 50) bei dem Einführen eine Endstellung erreichen, in welcher die Befestigungsmittel (98, 100), wenn die komprimierten Enden der Befestigungsmittel (98, 100) von einem Benutzer freigegeben werden, wieder aufgrund einer Kraft des komprimierten Federmittels (102), welches ein Teil der Funktionsmittelbefestigungsmechanismen (96, 97) ist, in ihre Ausgangsstellung vor dem Zusammendrücken durch einen Benutzer zurückkehren, wobei die Funktionsmittelbefestigungsmechanismen (96, 97) jeweils wenigstens ein Befestigungselement (116, 118, 120, 122) umfassen, welches als Verzahnung und/oder Kamm ausgebildet ist.

2. Chirurgisches Instrument nach Anspruch 1,
**dadurch gekennzeichnet, dass**
die Verzahnung wenigstens einen Zahn mit wenigstens zwei relativ zu einer sich senkrecht zu einer Einführrichtung (130) erstreckenden Ebene (132) geneigten Flächen (136, 130) aufweist.

3. Chirargisches Instrument nach einem der vorherigen Ansprüche,
**dadurch gekennzeichnet, dass**
wenigstens eine Stegeinheit (10, 12) zumindest ein lösbares Stegteil (48, 50) aufweist.

## Claims

1. Surgical instrument with one first and at least one second crosspiece module (10, 12), each of which comprises two crosspieces (48, 56, 50, 58) and at least one functional component (44, 46) and both of which are mounted movably with respect to each other, and with a first and a second handle component (14, 16), the first and second handle components (14, 16) being mounted movably with respect to each other and being intended for actuating the crosspiece modules (10, 12), wherein each of the two crosspiece modules (10, 12) has at least one detachable functional component (44, 46), and respectively with a functional component fastening mechanism (96, 97), which has at least one pivotably mounted fastener(98, 100), the functional component fastening mechanisms (96, 97) being provided for transmitting pulling and pressing movements and the upper crosspiece module (10) being translatively movable with respect to the lower crosspiece module (12), wherein fastening elements (120, 122) of the crosspieces (48, 50) are insertable into recesses (124, 126) of the crosspieces (56, 58) in an insertion direction (130), wherein insert chamfers (112, 114) of the crosspieces (56, 58) are provided, wherein, in case, due to the ends of the fasteners (98, 100) being pressed together, which is facilitated by a recess (108, 110) in each of the crosspieces (56, 58), said ends of the fasteners (98, 100) being respectively situated opposite the fastening elements (120, 122) and compressing a spring element (102) while being pressed together, the fastening elements (120, 122) are moved insufficiently away from one another, in that case said fastening elements (120, 122) are, due to contacting the insert chamfers (112, 114) during insertion of the crosspieces (48, 50) into the recesses (124, 126), pushed farther away from one another until the crosspieces (48, 50) arrive, in the course of being inserted, an end position, wherein the fasteners (98, 100) return, upon release of the compressed ends of the fasteners (98, 100) by a user, into their original position before being pressed together by a user, said return being once again due to a force of the compressed spring element (102), which is a part of the functional component fastening mechanisms (96, 97), wherein each of the functional component fastening mechanisms (96, 97) comprises at least one fastening element (116, 118, 120, 122) is implemented as a toothing and/or a comb.

2. Surgical instrument according to claim 1,
**characterized in that**
the toothing has at least one tooth with at least two surfaces (136, 138) which are inclined with respect to a plane extending perpendicularly to an insertion direction (130).

3. Surgical instrument according to one of the preceding claims,
**characterized in that**
at least one crosspiece unit (10, 12) has at least one detachable crosspiece (48, 50).

## Revendications

1. Instrument chirurgical avec une première et au moins une deuxième unité de traverse (10, 12) , chaque unité comportant deux pièces de traverse (48, 56, 50, 58) et au moins un élément fonctionnel (44,46), les unités de traverse (10, 12) étant montées mobiles l'une relativement à l'autre, et avec un premier et un deuxième élément de poignée (14, 16) qui sont montés mobiles l'un relativement à l'autre et sont prévus pour actionner les unités de traverse (10, 12), chacune des deux unités de traverse (10, 12) comportant au moins un élément fonctionnel (44, 46) détachable, et avec respectivement un mécanisme de fixation de l'élément fonctionnel (96, 97) qui contient respectivement au moins un dispositif de fixation (98, 100) qui est monté pivotant, où les mécanismes de fixation de l'élément fonctionnel (96, 97) sont prévus pour transmettre des mouvements de traction et pression, l'unité de traverse supérieure (10) étant montée mobile relativement à l'unité de traverse inférieure (12) par rapport à un mouvement translationnel, où des éléments de fixation (120, 122) des pièces de traverse (48, 50) sont introduisibles dans des échancrures (124, 126) des pièces de traverse (56, 58) en direction d' introduction (130), où des biseaux d'introduction (112, 114) des pièces de traverse (56,58) sont prévues, dans lequel instrument chirurgical, au cas où les éléments de fixation (120, 122) sont écartés insuffisamment l'un de l'autre à cause d'un bout d'un des dispositifs de fixation (98, 100) étant serré contre l'autre, les bouts des dispositifs de fixation (98, 100) étant opposés respectivement aux éléments de fixation (120, 122) et compressant au cours du serrement un dispositif de ressort (102), ledit serrement étant facilité grâce à des échancrures (108, 110) situées respectivement dans les pièces de traverse (56, 58), dans ce cas lesdits éléments de fixation (120, 122) sont plus écartés, du fait de qu'ils contactent les biseaux d'introduction (112, 114) au cours de l'introduction des pièces de traverse (48, 50) dans les échancrures (124, 126), jusqu'à ce que les pièces de traverse (48, 50) arrivent au cours de l'introduction à une position finale dans laquelle, lorsque les bouts serrés des dispositifs de fixation (98, 100) sont lâchés par un utilisateur, les dispositifs de fixation (98, 100) rentrent dans sa position originale d'avant le serrement par un utilisateur, autrefois grâce à une force du dispositif de ressort (102) compressé, qui est un part des mécanismes de fixation de l'élément fonctionnel (96, 97), chacun desdits mécanismes de fixation de l'élément fonctionnel (96, 97) comportant au moins un élément de fixation (116, 118, 120, 122) qui est formé comme dentelure et/ou crénelure.

2. Instrument chirurgical selon la revendication 1,
**caractérisé en ce que**
la dentelure comporte au moins une dent avec au moins deux surfaces inclinées (136, 138) relativement à un plan (132) s'étendant perpendiculaire à une direction d'introduction (130).

3. Instrument chirurgical selon l'une des revendications précédentes,
**caractérisé en ce qu'**
au moins une unité de traverse (10, 12) comporte au moins une pièce de traverse (48, 50) détachable.
